# EUROPEAN PATENT APPLICATION

(11) **EP 1 862 086 A1**
(43) Date of publication of application: **05.12.2007**
(21) Application number: 06725800.4
(22) Date of filing: 03.03.2006
(51) Int. Cl.: A41D 13/12, A61B 19/08

(54) **PROTECTIVE MEDICAL DEVICE FOR RESIDUE-PRODUCING TECHNIQUES**

(30) Priority: 04.03.2005 ES 200500503 U
(71) Applicant: Esquerdo Laib, Monica, 30570 San José de la Vega (Murcia) (ES)
(72) Inventor: Esquerdo Laib, Monica, 30570 San José de la Vega (Murcia) (ES)
(74) Representative: Carvajal y Urquijo, Isabel
(86) International application number: PCT/ES2006/000102
(87) International publication number: WO 2006/092460

(57) **Abstract**

The invention relates to a protective medical device which is intended for use in different techniques that can produce solid or liquid residues and splashes. The inventive equipment comprises a bag which is made from a preferably-disposable material that is suitably flexible, impermeable and resistant, which has undergone a series of cutting and pre-punching operations and which can incorporate self-adhesive areas, in order to produce an integral device from a single part, comprising the following components: an impermeable cloth which protects the patient; a container for draining off any residues produced; and an apron which protects the person performing the examination, which is equipped with fixing elements such as fastening straps and/or self-adhesive strips

## Description

### Object of the Invention

This Utility Model relates to a protective medical device for residue-producing techniques, providing to the function for which it is intended a series of advantages that will be described below in addition to other advantages inherent to its constitution.

Specifically, the described invention has as an object integrating in a single part in a simple and inexpensive manner, the elements necessary for performing a medical examination or practice, protecting at the same time the person performing the examination or practice, the patient and the environment, there being a container for collecting, channeling, and/or storing the possible residues produced, such as washings, tissues, fluids or organic residues, for the disposal or handling.

### Background of the Invention

In relation to the state of the art, it must be mentioned that in the sector there are currently different systems based on bags or containers for collecting residues, which are much more elaborate devices and therefore complicated to manufacture and expensive.

Furthermore, due to their features and very specific constitution, said devices are solely and exclusively intended for the specific use that they have been designed for.

It must be indicated that the applicant of this Utility Model is unaware of the existence of a protective medical device for residue-producing techniques similar to the one proposed by this invention which, due to its simplicity of shapes, at the same time provides the qualities of simplicity for its production, minimum manufacturing costs and maximum efficacy, advantageously allowing its use to perform any type of medical examination or practice.

### Explanation of the Invention

As mentioned above, with this invention a medical device has been conceived which is configured as a protective device with a simplified manufacture, but which has all the required and aforementioned features for performing different techniques that can entail solid or liquid residues and splashes, obtaining multiple advantages with its use due to its practicality, such that the aforementioned drawbacks derived from the technique are overcome.

Specifically, the protective medical device for residue-producing techniques object of the invention comprises a bag which is made from a preferably-disposable material that is suitably flexible, impermeable and resistant, which has undergone a series of cutting and pre-punching operations and which can incorporate self-adhesive areas, in order to produce from a single part an integral device comprising the following components:
- An impermeable cloth intended to protect the patient. Depending on the type of medical examination or practice to be performed, this cloth can be placed under or on the patient; in the event it is placed on the patient, the pre-punched area it is provided with is torn in order to thus, through the hole formed, perform the practice in question, keeping the patient isolated and protected from the technique and its residues. It must be mentioned that the mentioned pre-punched area can vary in different embodiments of the invention in its location, its shape, its size or its number, and there also may not even be a pre-punched area, depending on the needs of each case, it being possible in such case that it is the person performing the medical examination or practice who, when necessary, perforates the cloth with the aid of any cutting instrument, obtaining the necessary hole located in the suitable position for the practice in question. In addition, as mentioned above it is possible to use the impermeable cloth under the patient, in such case acting as a protective cloth, isolating the bed linens; in such case, if there is a pre-punched area, it is not torn, and the bed that is covered remains perfectly impermeable and protected. An area provided with fixing systems, such as a self-adhesive strip allows fixing the cloth to the bed or to the patient in order to prevent accidental slips.
- A container for draining off the residues produced during the operation for its subsequent storage, handling or disposal.
- And finally, an apron which protects the person performing the examination, also provided with fixing elements such as fastening straps and/or self-adhesive strips.

Maximum protection against possible splashes on both the medical personnel and the floor is thus obtained with an element as simple and inexpensive as a bag, because since such bag is made of a single part, it forms a single field which prevents residues from spilling into the environment, protection and isolation of the patient from the technique and the residues it may produce, as well as aiding in the task of disposing of or handling the residues produced, since such residues are directly stored in the container.

The protective medical device for residue-producing techniques of the invention accordingly represents a novel structure with structural and constitutional features that have been unknown until now for such purpose, which reasons, combined with its practical usefulness, are more than enough reasons to grant the exclusive right being sought.

### Description of the Drawings

To better interpret the invention, several drawings are attached to this specification which show, by way of non-limiting example, an embodiment of the integral protective medical device for residue-producing techniques object of the invention, according to the principles of the claims.

In said drawings:
Figure 1 shows a schematic perspective view of an example of the integral protective medical device for residue-producing techniques of the invention in its initial or folded stage.
Figure 2 also shows a schematic perspective view of the invention according to the depiction of Figure 1 in its open stage.
Figure 3 also shows a schematic perspective view of the invention according to the depiction of Figures 1 and 2, completely open and ready to be used.

### Description of an Embodiment of the Invention

In view of the discussed figures and according to the numbers used, an embodiment of the invention can be seen in such figures comprising the parts indicated below:
-1- Bag.
-2a- Transverse cuts.
-2b- Longitudinal cuts.
-2c- Vertical cut.
-3- Protective apron.
-4- Impermeable cloth.
-5- Container.
-6- Self-adhesive strip.
-6a- Self-adhesive strip of the area of the protective apron -3-.
-6b- Self-adhesive strip of the area of the impermeable cloth -4-.
-7- Fastening straps.
-8- Pre-punching.

As shown in the mentioned figures, the protective medical device for residue-producing techniques comprises a body in the shape of a bag -1- having a preferably rectangular configuration that is made of a suitably flexible, impermeable and highly resistant material.

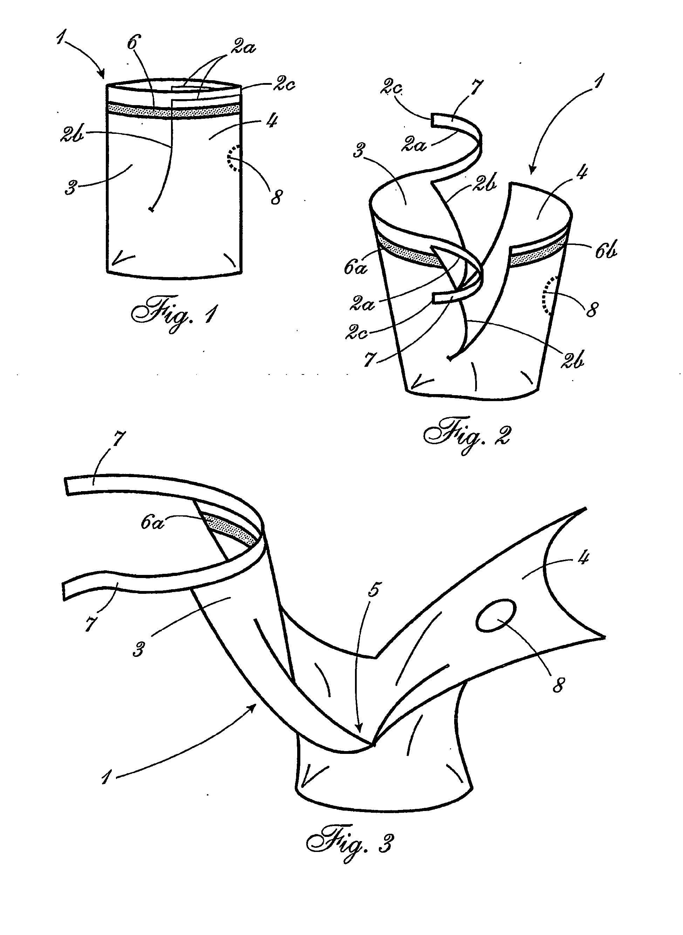

As can be seen in Figure 1, said bag -1- has in its upper part, close to the opening, respective transverse cuts -2a- approximately to the middle of said opening, and are bent downwards forming respective longitudinal cuts -2b- on both sides of the bag -1- and which extend to a certain height, having a slight curvature in their lower part, and a vertical cut -2c- going from the central part of the mentioned transverse cuts -2a- to the upper edge of the bag -1-.

The mentioned cuts -2a-, -2b- and -2c- split the bag -1- when it is opened, as shown in Figure 2, into two different and opposing parts, one of them forming the protective apron -3- intended for the person performing the medical examination or practice, and the other one forming the impermeable cloth -4- intended to isolate or the patient or protect the bed, as the case may be, forming a cavity between both parts and the bottom of the bag -1-, forming the container -5- intended to collect residues, as shown in Figure 3.

The invention also provides for the possibility that, thanks to conventional fixing means, both the part of the protective apron -3- and for the impermeable cloth -4- are provided with a self-adhesive strip -6- perimetrically located on the outer part close to the opening of the bag -1-, such that when it is opened and separated, area -6a-corresponding to the apron -3- allows the adhesive fixing of the latter to the health worker or person performing the examination, while area -6b- corresponding to the impermeable cloth -4- allows the adhesive fixing to the bed or to the patient. Said self-adhesive strip -6- will obviously be duly protected before it is used by means of wax paper or the like for example.

In addition, when the mentioned transverse cuts -2a- and vertical cut -2c- are separated from the bag -1-, they form respective fastening straps -7- useful for fixing the protective apron -3-, for example, to the neck or waist of the health worker, which straps can be used alone or together with the self-adhesive strip -6a-.

Finally, it is necessary to mention the existence, in the central part of the area corresponding to the impermeable cloth -4-, of an area in which pre-punching -8- can be applied, which pre-punching will preferably be circular-shaped, intended, as the case may be, for creating a hole for accessing the area of the patient to be treated, the latter thus being isolated and protected from possible splashes under the impermeable cloth -4-.

Having sufficiently described the nature of this invention, as well as the way of carrying it out to practice, it is hereby stated that it can be carried out to practice, within its essential nature, in other embodiments differing in detail from the embodiment indicated by way of example, with the materials and shapes which best adapt to each case, and which will be equally protected provided that its essential principle is neither altered, changed nor modified.

## Claims

1. A protective medical device for residue-producing techniques, of the type made of a single-use, flexible and impermeable and suitably resistant material, provided with fixing means such as self-adhesive strips (6) and/or fastening straps (7), **characterized by** being formed by a body in the shape of a bag (1) having a preferably rectangular configuration comprising in its upper part, close to the opening, respective transverse cuts (2a) extending approximately to the middle of said opening, and bent downwards, forming respective longitudinal cuts (2b) running on both sides of the bag (1) and extending to a certain height, having a slight curvature in their lower part and a vertical cut (2c) going from the central part of the mentioned transverse cuts (2a) to the upper edge of the bag -1-, in which the mentioned cuts (2a), (2b) and (2c) split the bag (1) when it is opened into two opposing parts forming the protective apron (3) and the impermeable cloth (4), respectively, the gap created between both of them and the bottom of the bag (1) forming a container (5) for collecting residues.

2. A protective medical device for residue-producing techniques according to the previous claim, **characterized in that** the part of the bag (1) corresponding to the impermeable cloth (4) allows its use indistinctly under the patient to protect the bed or on the patient to isolate him or her, and it can optionally be provided with a preferably circular-shaped pre-punched area (8) which, when torn, causes the creation of a hole for accessing the area of the patient to be treated for the option of using the impermeable cloth (4) on the patient.

3. A protective medical device for residue-producing techniques according to the previous claims, **characterized in that** the fixing means provided are: a self-adhesive strip (6) perimetrically located on the outer part close to the opening of the bag (1) in which, when opened and separated by the corresponding longitudinal cuts (2b), area (6a) corresponds to the protective apron (3) and allows the adhesive fixing thereof, and area (5b) corresponds to the impermeable cloth (4) and allows its adhesive fixing; and/or respective fastening straps (7) for the protective apron (3) formed by the transverse (2a) and vertical (2c) cuts when the bag (1) is separated.
